# EUROPEAN PATENT SPECIFICATION

(11) **EP 3 826 659 B1**
(45) Date of publication and mention of the grant of the patent: **19.06.2024**
(21) Application number: 19833946.7
(22) Date of filing: 10.07.2019
(51) Int. Cl.: A61K 38/00, A61K 38/10, A61P 31/04, A61K 31/70, A61K 31/53

(54) **IMMUNOMODULATOR FOR USE IN THE TREATMENT OF A BACTERIAL INFECTION**
IMMUNMODULATOR ZUR VERWENDUNG BEI DER BEHANDLUNG EINER BAKTERIELLEN INFEKTION
IMMUNOMODULATEUR DESTINÉ À ÊTRE UTILISÉ DANS LE TRAITEMENT D'UNE INFECTION BACTÉRIENNE

(30) Priority: 10.07.2018 US 201862695976 P
(43) Date of publication of application: 02.06.2021
(73) Proprietor: Rush University Medical Center, Chicago, IL 60612 (US)
(72) Inventor: SHAFIKHANI, Sasha, Hadi, Elmwood Park, IL 60707 (US)
(74) Representative: D Young & Co LLP
(86) International application number: PCT/US2019/041112
(87) International publication number: WO 2020/014298

(56) References cited:
- EP-B1- 1 778 833
- WO-A1-97/25350
- WO-A1-2009/067800
- WO-A1-2017/053327
- US-A- 4 916 118
- US-A- 6 165 459
- US-A1- 2012 128 622
- US-A1- 2016 346 354
- US-B1- 6 355 476
- US-B1- 6 399 078
- US-B1- 6 399 078
- US-B1- 6 586 430
- US-B2- 9 981 011
- MENTEN P ET AL: "Macrophage inflammatory protein-1", CYTOKINE & GROWTH FACTOR REVIEWS, ELSEVIER LTD, GB, vol. 13, 1 January 2002 (2002-01-01), pages 455-481, XP003000537, ISSN: 1359-6101, DOI: 10.1016/S1359-6101(02)00045-X
- DIPIETRO L A: "MIP-1 ALPHA AS A CRITICAL MACROPHAGE CHEMOATTRACTANT IN MURINE WOUND REPAIR", THE JOURNAL OF CLINICAL INVESTIGATION, B M J GROUP, GB, vol. 101, no. 8, 1 April 1998 (1998-04-01) , pages 1693-1698, XP002123772, ISSN: 0021-9738
- KOSTARNOY ALEXEY V. ET AL: "Topical Bacterial Lipopolysaccharide Application Affects Inflammatory Response and Promotes Wound Healing", JOURNAL OF INTERFERON AND CYTOKINE RESEARCH., vol. 33, no. 9, 1 September 2013 (2013-09-01), pages 514-522, XP055870700, US ISSN: 1079-9907, DOI: 10.1089/jir.2012.0108
- KÜHN KLAUS-DIETER ET AL: "Local antibiotic therapy", UNFALLCHIRURG, SPRINGER, BERLIN, DE, vol. 120, no. 7, 22 June 2017 (2017-06-22) , pages 561-572, XP036274054, ISSN: 0177-5537, DOI: 10.1007/S00113-017-0372-8 [retrieved on 2017-06-22]
- WOOD et al.: "Pro-Inflammatory Chemokine CCL2 (MCP-1) Promotes Healing in Diabetic Wounds by Restoring the Macrophage Response", PLoS One, vol. 9, no. 3, 11 March 2014 (2014-03-11), pages 1-8, XP055224734, DOI: 10.1371/journal.pone.0091574
- Leichtle Anke ET AL: "CC Chemokine Ligand 3 Overcomes the Bacteriocidal and Phagocytic Defect of Macrophages and Hastens Recovery from Experimental Otitis Media in TNF-/- Mice", The Journal of Immunology, vol. 184, no. 6, 15 March 2010 (2010-03-15), pages 3087-3097, XP93029426, US ISSN: 0022-1767, DOI: 10.4049/jimmunol.0901167 Retrieved from the Internet: URL:https://watermark.silverchair.com/ji_0 901167.pdf?token=AQECAHi208BE49Ooan9kkhW_E rcy7Dm3ZL_9Cf3qfKAc485ysgAABLQwggSwBgkqhki G9w0BBwagggShMIIEnQIBADCCBJYGCSqGSIb3DQEHA TAeBglghkgBZQMEAS4wEQQMwzA_yrKOmEmycdZDAgE QgIIEZw3EEsJKOn2uHIwzpNznHGZhXe0u7yOXGEtpr LI0k5pfGYh2LO0XM0C95LudOBvB6iSx-EGGa0sj-jx SuXDNOn5P1>

## Description

### RELATED APPLICATIONS

### FEDERALLY SPONSORED RESEARCH OR DEVELOPMENT

This invention was made with government support under grant number RO1DK107713 awarded by the National Institute of Diabetes and Digestive and Kidney Diseases. The government has certain rights in the invention.

### REFERENCE TO APPENDIX [CD ROM/Sequence listing]

The instant application contains a Sequence Listing which has been submitted electronically in ASCII format. The ASCII copy, created on July 9, 2019, is named 14904-483 Sequence listing_ST25.txt and is 26 KB in size.

### BACKGROUND

### 1. Technical Field text

The present disclosure pertains to methods and compositions for controlling infection and stimulating healing in normal and diabetic wounds.

### 2. Background Information

Despite many advances in infection control practices - including improved operating room ventilation, barriers, sterilization methods, improved surgical techniques, and administration of antimicrobial prophylaxis - infections remain a significant cause of morbidity, prolonged hospitalization, and death (Mathers et al., 2006, Organization, 2010, Rubinstein *et al.,* 2011, Lim *et al.,* 2012). The annual costs for Healthcare Associated Infections (HAls) is astronomical and increasing every year, with surgical site infections (SSI) contributing the most to overall costs (-33.7% of the total), approximately $3.4 billion in US and between $3.5 to $10 billion in Europe (Zimlichman *et al.,* 2013, Anderson *et al.,* 2014).

Although, antibiotics have saved millions of lives over the past century, their use is not without its problems. First, there is currently no antibiotic on the market that is effective against all bacterial pathogens, thus they are limited in their activity spectra. Second, even short-term antibiotic use has been associated with the emergence of antibiotic resistance (Kinch *et al.,* 2014, Blair *et al.,* 2015, Ventola, 2015, Korpela *et al.,* 2016, Langdon *et al.,* 2016), which heightens the prospect of currently treatable infections becoming unmanageable pathogens with pandemic potential. Further, exacerbating this situation of resistance is the dwindling investments in discovery and development of new antibiotics to face this ever-growing challenge (Cooper *et al.,* 2011). Third, antibiotics have many undesirable and dangerous side-effects; including - but not limited to -- nephrotoxicity, hepatotoxicity, mitochondrial damage, and dysbiosis in gut microbiome which itself has been associated with obesity, diabetes, and immunological and neurological diseases such as Parkinson disease (Grill *et al.,* 2011, Singh *et al.,* 2014). Fourth, prophylaxis antibiotic is associated with increased risk of Clostridium difficile infection (Balch *et al.,* 2017). Addition of rCCL3 alone or in combination with rTNF restores phagocytosis and killing by TNF-deficient macrophages to that of unstimulated wild-type macrophages. *In vivo* administration of rCCL3 to animals deficient in TNF fully restores the ability to control OM due to NTHi (*Haemophilus influenzae*), whereas a CCL3-blocking Ab impaired the ability of wild-type mice to recover from OM (Leichtle et al. 2010, The Journal of Immunology, 184: 3087-3097). Therefore, new approaches, including antibiotic-free approaches or antibiotic combination approaches, are needed to address infection.

### BRIEF SUMMARY

The invention is set out in the appended set of claims.

### BRIEF DESCRIPTION OF THE DRAWINGS

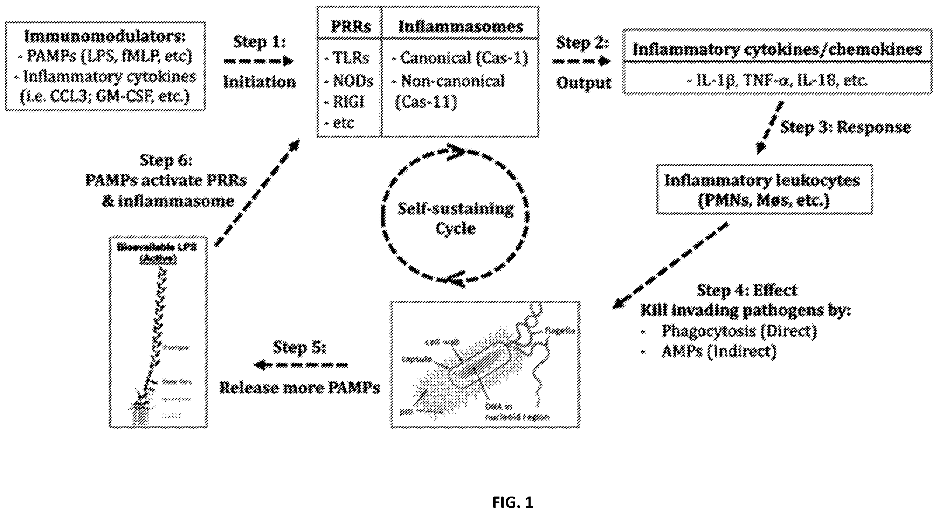

FIG. 1 illustrates how immunomodulators can be used to enhance infection control.

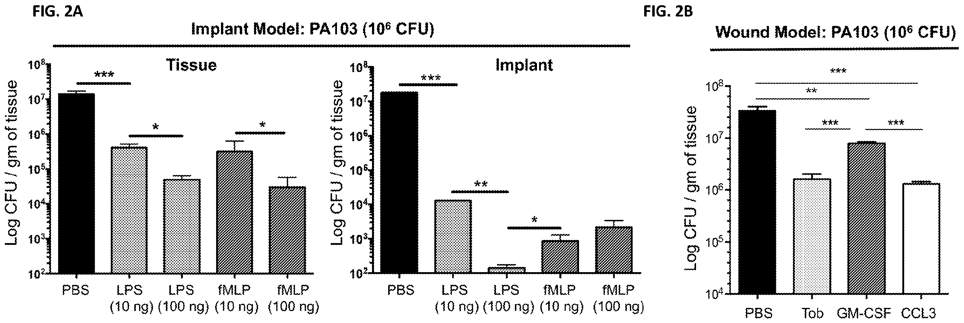

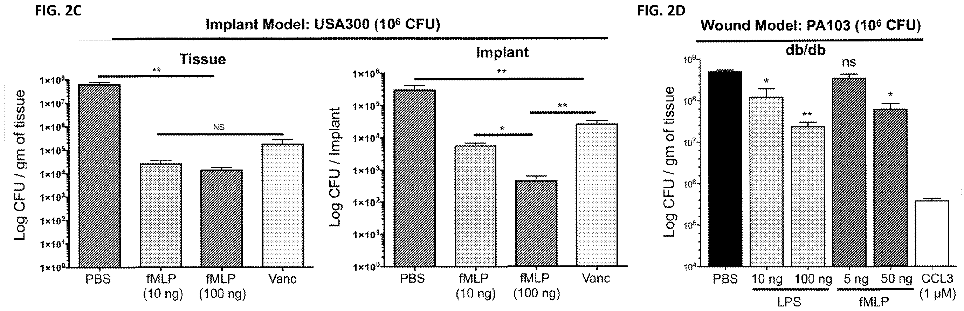

FIGS. 2A-2D illustrates that immunomodulators are effective in controlling infection. The implant model of infection in C57B mice was used for 10⁶ PA103 (FIG. 2A) or 10⁶ USA300 MRSA (FIG. 2C). The wound model of infection was used for 10⁶ PA103 in C57BL mice (FIG. 2B) or diabetic db/db mice (FIG. 2D). Infection was carried out at the time of surgery by adding bacteria directly to the surgical sites. Immunomodulators, namely, LPS (10 ng or 100 ng), fMLP (10 ng or 100 ng), CCL3 (1µg) were added topically at the surgical sites at the time of surgery & infection. GM-CSF (10µg/kg), tobramycin (Tob at 3.5mg/kg), and vancomycin (40 mg/kg) were added systemically by i.p. injection 1h before surgical implantation and infection. Infection levels were determined 24h following infection (N> 4 animals/group; NS= not significant, * *p*<0.01, ** *p*<0.001, *** *p*<0.0001 One-way ANOVA).
FIGS. 3A and 3B illustrate that CCL3 treatment enhances antimicrobial defenses against P. *aeruginosa* in a normal wound in the presence or absence of prophylaxis antibiotic. N=4; Significance determined as compared to PBS *<0.05, **p<0.01, ***p<0.001.
FIGS. 4A-4C illustrate that PAMPs enhance P. *aeruginosa* PA103 10⁶ infection control in a normal wound. (4A-LPS, 4B-fMLP, 4C-tobramycin) N=4; Significance determined as compared to PBS *<0.05, **p<0.01, ***p<0.001, ****p<0.0001.

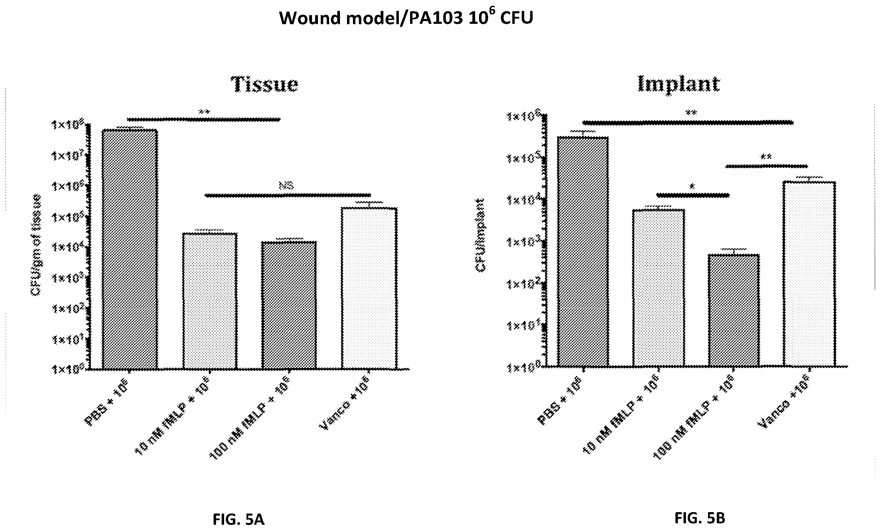

FIGS. 5A and 5B illustrate that PAMPs are effective in controlling S. *aureus* surgical site infection as prophylactic antibiotic standard therapy. S. *aureus* (10⁶ CFU). S. *aureus* titers after 1 day. Significance determined as compared to PBS *<0.05, **p<0.01, ***p<0.001, ****p<0.0001.
FIGS. 6A and 6B illustrate prophylaxis antibiotic standard therapy +/immunomodulator IL-1β therapy to control P. *aeruginosa* (6A) or MRSA (6B) infection. N=4; Significance determined as compared to PBS *<0.05, **p<0.01, ***p<0.001, ****p<0.0001.

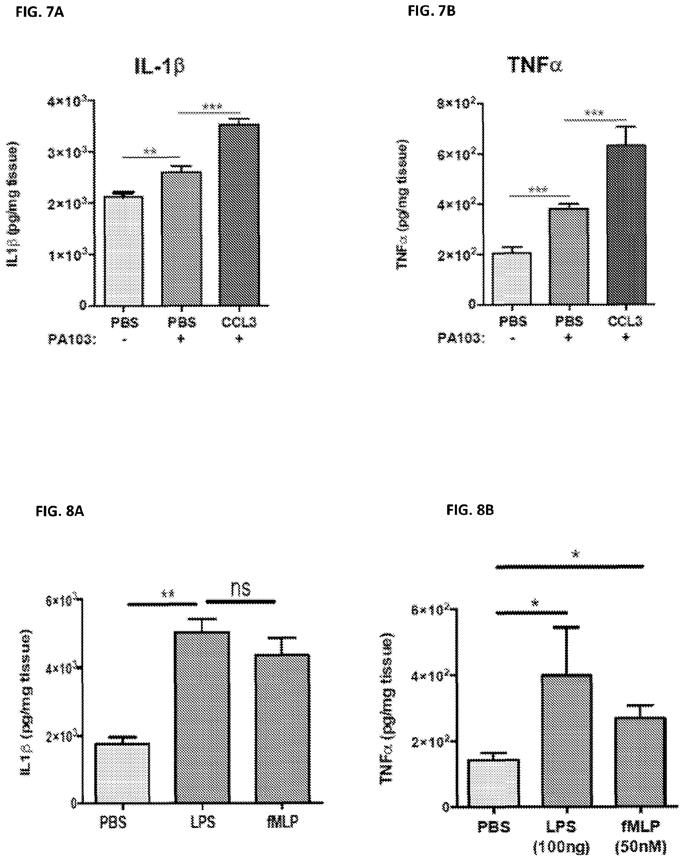

FIGS. 7A and 7B illustrate that CCL3 treatment increases the production of inflammatory cytokines in a normal wound. N=4; Significance determined as compared to PBS *<0.05, **p<0.01, ***p<0.001.
FIGS. 8A and 8B illustrate that LPS and fMLP treatment increases the production of inflammatory cytokines in a normal wound. N=4; LPS (10 ng) and fMLP (100 nM) directly added to wounds at the time of infection after wounding. Significance determined as compared to PBS *<0.05, **p<0.01.

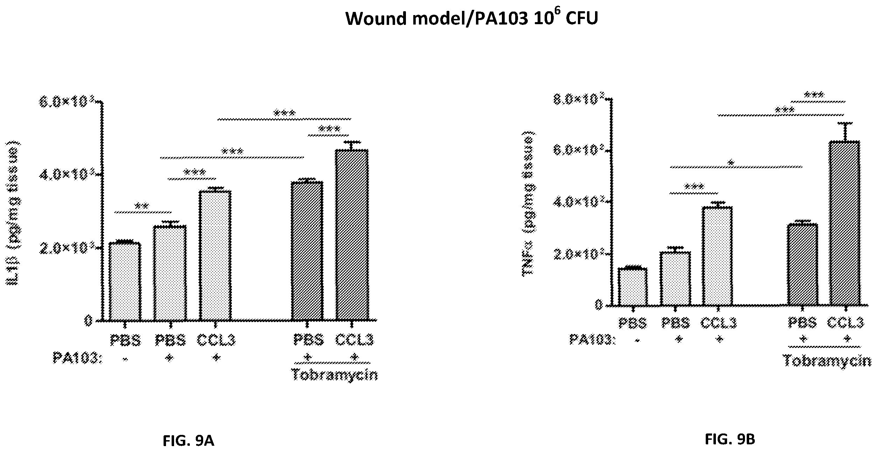

FIGS. 9A and 9B illustrate the impact of CCL3 +/- tobramycin on inflammatory cytokines. N=4; Significance determined as compared to PBS *<0.05, **p<0.01, ***p<0.001, ****p<0.0001.
FIGS. 10A-10D illustrate that immunomodulatory treatment enhances leukocyte migration and neutrophil activation in a normal wound. (10A, 10B- CCL3, 10C-LPS, fMLF, 10D-GM-CSF), N=4; Significance determined as compared to PBS *<0.05, **p<0.01, ***p<0.001.
FIGS. 11A-11C illustrate that CCL3 increases infection control (11A), collagen deposition (11B) and enhances wound healing (11C) in diabetic wounds. P. *aeruginosa* PA103 10³ N=4; Significance determined as compared to PBS *<0.05, **p<0.01, ***p<0.001.

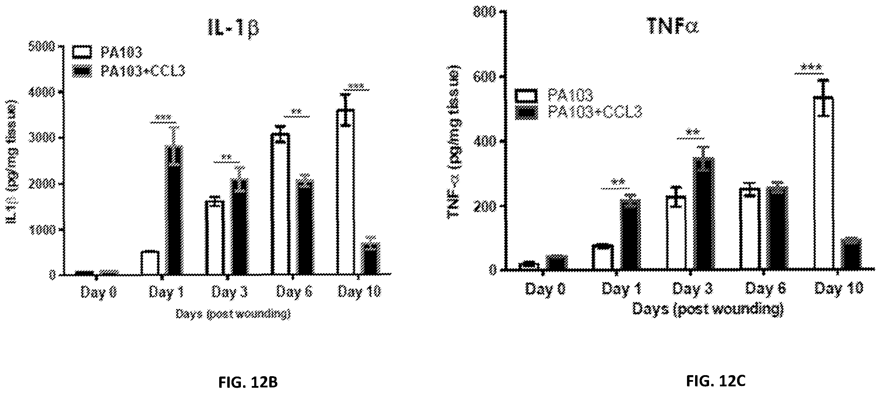

FIGS. 12A-12C illustrate that CCL3 does not lead to neutrophilia (12A) or persistent inflammatory responses (12B IL-1 β, 12C TNFα) in infected diabetic wounds. P. *aeruginosa* PA103 10³ N=4; Significance determined as compared to PBS *<0.05, **p<0.01, ***p<0.001.

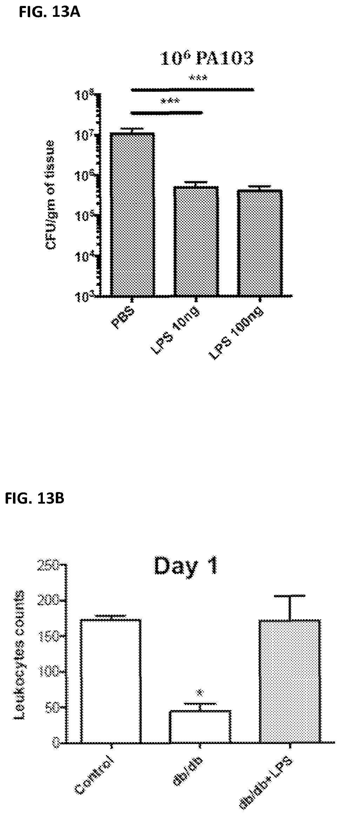

FIG. 13A illustrates that TLR4 ligand increases antimicrobial defenses in diabetic wounds against P. aeruginosa and FIG. 13B TLR4 ligand stimulates inflammatory responses in diabetic wounds. N=4; Significance determined as compared to PBS *<0.05, **p<0.01, ***p<0.001.
FIG. 14 illustrates CCL3 enhances wound healing in diabetic uninfected wounds.

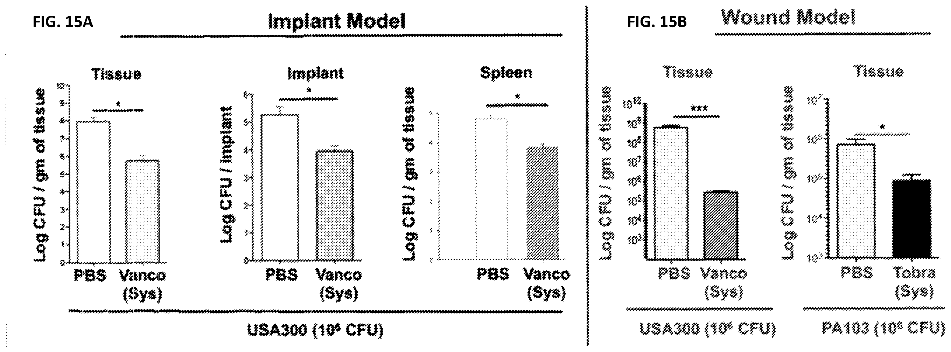

FIGS. 15A and 15B illustrate that prophylaxis antibiotic therapies are effective in controlling surgical site infections in vivo. (15A) C57BL/6 mice received 40mg/kg prophylaxis (Sys) vancomycin (Vane) or 3.5mg/kg tobramycin (Tobra) by i.p. injection. (15A) 1h after antibiotic administration, 10⁶ CFU of USA300 was added to implant after insertion into facia (Implant Model). (15B) 1h after antibiotic administration, 10⁶ CFU of USA300 or 10⁶ CFU PA103 (Pa) were added full-thickness wounds in of C57BL/6 (Wound Model). Infection levels at implant surgical site (Tissue & Implant) or a distant site (Spleen), or wound tissues were assessed 24h following infection. (N> 4 animals/group, * *p*<0.01, *** *p*<0.0001, One-way ANOVA).

### DETAILED DESCRIPTION

The embodiments disclosed below are not intended to be exhaustive or to limit the scope of the disclosure to the precise form in the following description. Rather, the embodiments are chosen and described as examples so that others skilled in the art may utilize its teachings. Unless otherwise defined, all technical and scientific terms used herein have the same meaning as commonly understood by one of ordinary skill in the art to which this disclosure pertains.

The uses of the terms "a" and "an" and "the" and similar references (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example") provided herein, is intended merely to better illuminate the disclosure and does not pose a limitation on the scope unless otherwise claimed. No language in the specification should be construed as indicating any non-claimed element as essential to the practice of the embodiments.

As used herein, the term "amount" refers to "an amount effective" or "therapeutically effective amount" of a composition, e.g., an immunomodulator, to achieve a beneficial or desired prophylactic or therapeutic result, including clinical results. A "therapeutically effective amount" of a composition may vary according to factors such as the disease state, age, sex, and weight of the individual, and the ability of the antibody or peptide to elicit a desired response in the individual. A therapeutically effective amount is also one in which any toxic or detrimental effects of the virus or transduced therapeutic cells are outweighed by the therapeutically beneficial effects. The term "therapeutically effective amount" includes an amount that is effective to "treat" a subject (e.g., a patient). When a therapeutic amount is indicated, the precise amount of the compositions of the present disclosure to be administered may be determined by a physician with consideration of individual differences in age, weight, extent of infection, and condition of the patient (subject).

### Immunomodulators

Immunomodulators as used herein include Pathogen Associated Molecular Pattern Molecules (PAMPs) and/or pro-inflammatory cytokines and chemokines. Immunomodulators include any agent that mobilizes neutrophils to an infection or wound site in a subject, including surgically induced wounds that may or may not be infected. To demonstrate different types of immunomodulators that may be used to mobilize neutrophils, immunomodulators have been assigned to four arbitrary classes based on the activity of each class obtained from a review of the current literature. In this disclosure, class 1 immunomodulators are defined as those that function primarily as chemoattractants for phagocytic leukocytes. To represent class 1, Chemokine (C-C motif) ligand 3 (CCL3) (a.k.a macrophage inflammatory protein 1-alpha, MIP-1α) immunomodulator was chosen which has been shown to recruit and activate phagocytic leukocytes by primarily engaging their chemotaxis receptors (CCR1 and CCR5). Wild type human CCL3 with a 23 amino acid signal sequence that may be cleaved is shown in SEQ ID NO: 1 (UniProtKB - P10147). In some aspects, CCL3 as used herein includes modifications to CCL3 to prevent or reduce multimer formation beyond certain stages (for example a dodecamer) so that the resulting low molecular weight monomers (or oligomers) have improved solution properties. Exemplary modifications may be found in U.S. Patent 6,057,123. In some aspects, the CCL3 immunomodulator may be BB-10010 (British Biotech Pharmaceuticals, Ltd.) BB-10010 includes a modification of Asp26>Ala in the mature form of human CCL3 (SEQ ID NO: 2). (BB-10010 is also described in Hunter et al. 1995, Blood, Vol. 86, No. 12, pp. 4400-4408.) Other non-limiting examples of class 1 include agents that target CCR1, CXCR1, or CXCR2 and mobilize neutrophils. Some of the examples of immunomodulators that target human CXCR1 or CXCR2 receptors, include IL-8 (SEQ ID NO; 4, with signal peptide, UniProtKB - P10145) or C-X-C motif ligand 1 (CXCL1/MGSA) (SEQ ID NO: 5, precursor), or neutrophil-activating peptide-2 (NAP-2) (SEQ ID NO: 6, precursor). Class 2 is defined herein as those immunomodulators that increase pro-inflammatory cytokines by engaging Pattern Recognition Receptors (PRRs) and/or inflammasomes. To represent class 2, lipopolysaccharide (LPS) was chosen, which is a PAMP immunomodulator that triggers inflammatory responses primarily through Toll like receptor 4 (TLR4) and secondarily through TLR2 and non-canonical caspase-11 inflammasome. Other non-limiting examples of class 2 include Lipoteichoic acids (activates TLR2 receptor); Pam2CSK4 (activator of TLR2/TLR6); Polyinosinic-polycytidylic acid (poly(I:C) (activates TLR3); & Recombinant Flagellin from S. typhimurium which activates TLR5. Class 3 as defined herein are those immunomodulators that exhibit features of classes 1 & 2. To represent class 3, N-Formylmethionyl-leucyl-phenylalanine (fMLP) (PubChem CID: 443295) was chosen which is both a potent chemoattractant and an activator of immune leukocytes, targeting them through FPR primary receptor, but it has also been implicated in the activation of TLR2 and TLR4 PRRs. Class 4 are defined herein as those immunomodulators that primarily promote the production and/or maturation of myeloid cells (i.e., neutrophils and macrophages). To represent class 4, Granulocyte-macrophage colony-stimulating factor (GM-CSF) (SEQ ID NO: 3, UniProtKB - P04141, precursor) was chosen which is shown to promote survival, clonal expansion, and differentiation of hematopoietic progenitor myeloid cells. GM-CSF is approved by the FDA for systemic use to boost myeloid production and to accelerate neutrophil recovery in neutropenic patients with acute lymphoblastic leukemia, non-Hodgkin's lymphoma, and Hodgkin's disease undergoing autologous stem cell transplantation; in older adult patients with AML, following induction chemotherapy; in patients undergoing allogeneic stem cell transplantation from HLA-matched related donors; and in patients in whom engraftment fails after allogeneic or autologous stem cell transplantation. has also been implicated in the activation of TLR2 and TLR4 PRRs. Other non-limiting examples of class 4 include Granulocyte-colony stimulating factor (G-CSF) (SEQ ID NO: 7, precursor; or Monocyte-colony stimulating factor (M-CSF) (SEQ ID NO: 8, precursor) or IL-13 (SEQ ID NO: 9, precursor) or IL-6 (SEQ ID NO: 10, precursor).

Pro-inflammatory cytokines include but are not limited to interleukin-1 (IL-1, for example, IL-1β, SEQ ID NO: 11, precursor), IL-12 (SEQ ID NO: 12, precursor), and IL-18 (SEQ ID NO: 13, precursor), tumor necrosis factor alpha (TNF-α) (SEQ ID NO: 14, precursor), interferon gamma (IFNγ) (SEQ ID NO: 15, precursor), and granulocyte-macrophage colony stimulating factor (GM-CSF). Chemokines are chemoattractant cytokines. One example of a chemokine that may be used as an immunomodulator is CCL3. The wild type human amino acid sequences provided herein are from the Uniprot database and may be further processed to the mature form that may be used for the immunomodulator delivered to the subject.

The immunomodulators may be provided as isolated polypeptides, free from combination with other cellular components unless specifically combined for administration. One, two, three, four, five or more immunomodulators may be delivered to the subject. Isolated CCL3 for use according to the invention may be administered to the subject alone, in combination with any of LPS, fMLP, GM-CSF or other class 1, 2, 3 and/or 4 immunomodulators, and/or in combination with an antibiotic. The antibiotic may be selected according to the infectious pathogen and suitable treatment known by one of skill in the art.

In some aspects, immunomodulators as described herein can be administered to a subject in need of the treatment via a suitable route. The immunomodulators can be administered to a subject in need of the treatment directly or indirectly (e.g., via one or more expression vectors adapted for expressing the immunomodulator). Such an expression vector can be constructed by inserting one or more nucleotide sequences of the immunomodulator into a suitable expression vector, in which the immunomodulator sequence(s) are in operable linkage with a suitable promoter.

### Pharmaceutical Compositions

Formulations of therapeutic and diagnostic agents can be prepared by mixing with physiologically acceptable carriers, excipients, or stabilizers in the form of, e.g., lyophilized powders, slurries, aqueous solutions, lotions, or suspensions (see, e.g., Hardman et al., (2001) Goodman and Gilman's The Pharmacological Basis of Therapeutics, McGraw-Hill, New York, N.Y.; Gennaro (2000) Remington: The Science and Practice of Pharmacy, Lippincott, Williams, and Wilkins, New York, N.Y.; Avis, et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman, et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, N.Y.).

Selecting an administration regimen for a therapeutic depends on several factors, including the serum or tissue turnover rate of the entity, the level of symptoms, the immunogenicity of the entity, and the accessibility of the target cells in the biological matrix. In certain embodiments, an administration regimen maximizes the amount of therapeutic delivered to the patient consistent with an acceptable level of side effects. Accordingly, the amount of biologic delivered depends in part on the particular entity and the severity of the condition being treated. Guidance in selecting appropriate doses of antibodies, cytokines, and small molecules are available (see, e.g., Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, N.Y.; Bach (ed.) (1993) Monoclonal Antibodies and Peptide Therapy in Autoimmune Diseases, Marcel Dekker, New York, N.Y.; Baert et al, (2003) New Engl. J. Med. 348:601-608; Milgrom et al, (1999) New Engl. J. Med. 341 : 1966-1973; Slamon et al, (2001) New Engl. J. Med. 344:783-792; Beniaminovitz et al, (2000) New Engl. J. Med. 342:613-619; Ghosh et al, (2003) New Engl. J. Med. 348:24-32; Lipsky et al, (2000) New Engl. J. Med. 343: 1594-1602).

Determination of the appropriate dose is made by the clinician, e.g., using parameters or factors known or suspected in the art to affect treatment or predicted to affect treatment. Generally, the dose begins with an amount somewhat less than the optimum dose and it is increased by small increments thereafter until the desired or optimum effect is achieved relative to any negative side effects. Important diagnostic measures include those of symptoms of, e.g., the inflammation or level of inflammatory cytokines produced.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors known in the medical arts. Compositions comprising one or more immunomodulators can be provided by doses at intervals of, e.g., one day, one week, or 1-7 times per week or by continuous infusion. Doses may be provided topically, intravenously, subcutaneously, orally, nasally, rectally, intramuscular, intracerebrally, or by inhalation. A specific dose protocol is one involving the maximal dose or dose frequency that avoids significant undesirable side effects.

In some aspects, the immunomodulator for use according to the invention may be topically administered to a subject or an implant. The method described herein may include administering about 0.1 micrograms per square millimeter (µg/mm²) to about 10 µg/mm² of the immunomodulator to the infected site, wound, surface area of an implant or other application or administering about 0.2 µg/mm² to about 8 µg/mm² of the immunomodulator, or administering about 0.25 µg/mm² to about 5 µg/mm² of the immunomodulator, or administering about 0.3 µg/mm² to about 3 µg/mm² of the immunomodulator, or administering about 0.3 µg/mm² of the immunomodulator, or any value in between the endpoints of the recited ranges.

A total weekly dose may be at least 0.05 µg/kg body weight, at least 0.2 µg/kg, at least 0.5 µg/kg, at least 1 µg/kg, at least 10 µg/kg, at least 100 µg/kg, at least 0.2 mg/kg, at least 1.0 mg/kg, at least 2.0 mg/kg, at least 10 mg/kg, at least 25 mg/kg, at least 30 mg/kg, at least 40 mg/kg or at least 50 mg/kg (see, e.g., Yang et al, (2003) New Engl. J. Med. 349:427-434; Herold et al, (2002) New Engl. J. Med. 346: 1692-1698; Liu et al, (1999) J. Neurol. Neurosurg. Psych. 67:451-456; Portielji et al, (2003) Cancer Immunol. Immunother. 52: 133-144).

An effective amount for a particular patient may vary depending on factors such as the condition being treated, the overall health of the patient, the method route and dose of administration and the severity of side effects (see, e.g., Maynard et al., (1996) A Handbook of SOPs for Good Clinical Practice, Interpharm Press, Boca Raton, Fla.; Dent (2001) Good Laboratory and Good Clinical Practice, Urch PubL, London, UK).

The route of administration may be by, e.g., topical or cutaneous application, injection or infusion by intravenous, intraarticular, intraperitoneal, intracerebral, intramuscular, intraocular, intraarterial, intracerebrospinal, intralesional, or by sustained release systems or an implant (see, e.g.,Sidman et al., (1983) Biopolymers 22:547-556; Langer et al., (1981) J. Biomed. Mater. Res. 15: 167-277; Langer (1982) Chem. Tech. 12:98-105; Epstein et al, (1985) Proc. Natl. Acad. Sci. USA 82:3688-3692; Hwang et al., (1980) Proc. Natl. Acad. Sci. USA 77:4030-4034; U.S. Pat. Nos. 6,350,466 and 6,316,024). Where necessary, the composition may also include a solubilizing agent and a local anesthetic such as lidocaine to ease pain at the site of the injection. In addition, pulmonary administration can also be employed, e.g., by use of an inhaler or nebulizer, and formulation with an aerosolizing agent. See, e.g., U.S. Pat. Nos. 6,019,968, 5,985,320, 5,985,309, 5,934,272, 5,874,064, 5,855,913, 5,290,540, and 4,880,078; and PCT Publication Nos. WO 92/19244, WO 97/32572, WO 97/44013, WO 98/31346, and WO 99/66903.

Methods for co-administration or treatment with a second therapeutic agent, e.g., an antibiotic by way of non-limiting example, are known in the art (see, e.g., Hardman et al., (eds.) (2001) Goodman and Gilman's The Pharmacological Basis of Therapeutics, IO.sup.th ed., McGraw-Hill, New York, N.Y.; Poole and Peterson (eds.) (2001) Pharmacotherapeutics for Advanced Practice: A Practical Approach, Lippincott, Williams & Wilkins, Phila., Pa.; Chabner and Longo (eds.) (2001) Cancer Chemotherapy and Biotherapy, Lippincott, Williams & Wilkins, Phila., Pa.). An effective amount of therapeutic may decrease the symptoms by at least 10%; by at least 20%; at least about 30%>; at least 40%>, or at least 50%.

Additional therapies (e.g., prophylactic or therapeutic agents), which can be administered in combination with the agent described herein may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the antibodies or fragments thereof of the invention. The two or more therapies may be administered within one same patient visit.

The immunomodulator for use according to the invention may be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, i.e., the cycle in order to reduce the development of resistance to one of the therapies, to avoid or reduce the side effects of one of the therapies, and/or to improve the efficacy of the therapies.

In certain embodiments, the agents can be formulated to ensure proper distribution in vivo. For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., Ranade, (1989) J. Clin. Pharmacol. 29:685). Exemplary targeting moieties include folate or biotin (see, e.g., U.S. Pat. No. 5,416,016 to Low et al); mannosides (Umezawa et al, (1988) Biochem. Biophys. Res. Commun. 153: 1038); antibodies (Bloeman et al, (1995) FEBS Lett. 357: 140; Owais et al., (1995) Antimicrob. Agents Chemother. 39: 180); surfactant protein A receptor (Briscoe et al, (1995) Am. J. Physiol. 1233: 134); p 120 (Schreier et al, (1994) J. Biol. Chem. 269:9090); see also K. Keinanen; M. L. Laukkanen (1994) FEBS Lett. 346: 123; J. J. Killion; I. J. Fidler (1994) Immunomethods 4:273.

The therapies (e.g., prophylactic or therapeutic agents) of the combination therapies can be administered to a subject concurrently. The term "concurrently" is not limited to the administration of therapies (e.g., prophylactic or therapeutic agents) at exactly the same time, but rather it is meant that a pharmaceutical composition comprising antibodies or fragments thereof described herein are administered to a subject in a sequence and within a time interval such that the antibodies of the invention can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a subject within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

### Therapeutic Uses

In some aspects, the immunomodulators may be used to control infection at surgical sites or in wounds. Surgical site infections (SSI) include but are not limited to superficial incisional infections, infections of the deep incision space and organ space infections. In some aspects, immunomodulators may be used to enhance the effectiveness of antibiotic therapy in controlling infection. In some aspects, immunomodulators may be used in normal subjects or diabetic subjects..

In some aspects, the immunomodulator may be topically applied. The topical application may be in a gel formulation or other topical application. In some aspects, the topical application of the immunomodulator may be at a surgical site during surgery. In some aspects, the immunomodulator may be at a surgical site during surgery in combination with prophylactic antibiotic therapy. In some aspects, an immunomodulatory may be topically coated or impregnated into an implant prior to surgical implantation. In some aspects, the immunomodulator may be topically applied to an infected diabetic wound after debridement to control infection and to stimulate healing. In some aspects, the immunomodulators may be administered systemically to boost infection control. In some aspects, the system administration may be by subcutaneous injection.

### Immunomodulator-based approaches in controlling surgical site infections

In recent years, harnessing the body's own immune system to battle cancer by various immunotherapies, such as checkpoint inhibitors, has revolutionized treatment against many cancers for which there had been no viable options prior. Immunomodulators may be used to achieve similar success in controlling SSIs as cancer immunotherapies, particularly in lieu of the fact that pathogens are far more dissimilar from their host cells than cancer and normal cells are. Harnessing host innate immune responses by immunomodulators to control SSIs has many advantageous over prophylaxis antibiotics, though they may also be used with antibiotics in combination therapy to enhance infection control. 1) It is highly unlikely for a pathogen to develop resistance to all antimicrobial weapons that the immune system has at its disposal, including; phagocytosis, bursts of reactive oxygen species (ROS), hypochlorous acid (HOCI), neutrophil and macrophage extracellular traps (NET & MET), and antimicrobial peptides (AMPs), which are our own natural antibiotics with diverse structures and activities against viral, fungal, and bacterial pathogens. This also means that immunomodulator-based approaches would not be empirically based because, once mobilized to the site of infection, immune system may be effective in controlling majority of infections regardless of their origin (bacterial, fungal, or viral). 2) Many pathogens, (including P. *aeruginosa* and *S*. *aureus*), possess stealth virulence strategies that allow them to establish infection even in immunocompetent individuals by dampening host's immune responses. Immunomodulators could potentially overcome at least some of these stealth strategies and enhance tissue's defenses against invading pathogens by mobilizing immune responses to the site of these stealth pathogens. 3) Immunomodulators may have less undesirable side-effects and may be safer than antibiotics, which as discussed above, have many undesirable and dangerous side-effects. For example, in some aspects, topical delivery at the site may be used and it is unlikely that topical use of immunomodulators would result in bacterial resistance, gut dysbiosis, or organ damage (side-effects associated with use antibiotics), given that the immunomodulators are primarily produced in our body (except PAMPs), and the route of delivery, (topical versus systemic use that is used for prophylaxis antibiotics). Other routes of administration for the immunomodulators may also provide advantages over antibiotics. Further, immunomodulators may be used in combination with antibiotics.

Immunomodulators are effective in controlling surgical site infections against MRSA and P. *aeruginosa.*

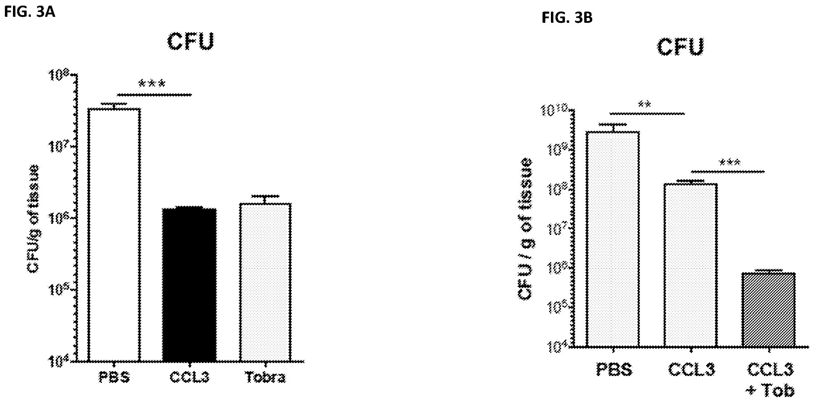

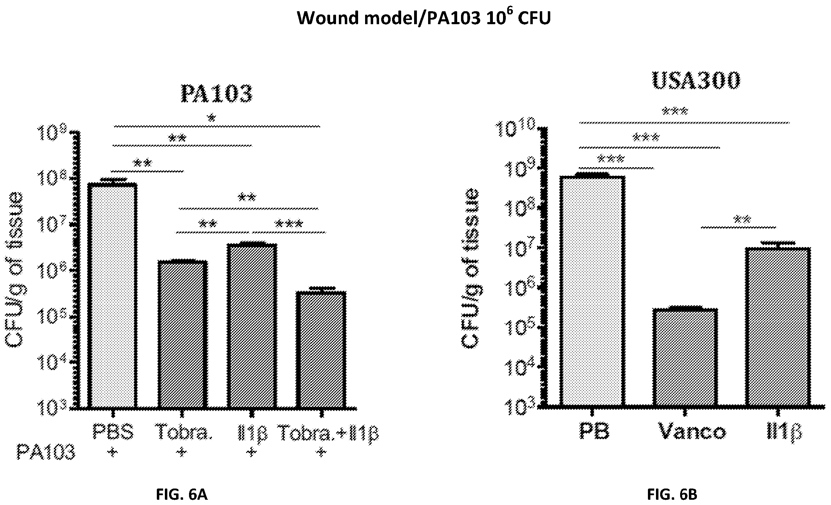

We assessed these immunomodulators for their ability to control wound and/or implant SSIs in normal (C57BL/6) or diabetic (db/db) mice. We acknowledge that diabetic individuals are not as immunocompromised as other immunocompromised patient groups. However, we included db/db animals because impaired neutrophil functions and immune dysregulation have been shown to render both human diabetics and db/db mice highly vulnerable to infection. db/db mice are extensively used as type 2 diabetic animal. All immunomodulators were added locally at the surgical sites at the time of infection, except GM-CSF (10µg/kg) which was administered systemically by i.p. injection 1h prior to surgery per its FDA-approved route of delivery. Our data from an implant model infected with PA103 (a clinical isolate of P. aeruginosa that causes serions infection and wound damage in diabetic wounds) indicated that topical treatments with fMLP or LPS significantly reduced P. *aeruginosa* infection levels on both the implants and tissues surrounding implants in a dose-dependent manner (Fig 2A, N>4 animals/group; * *p*<0.01, ** *p*<0.001, *** *p*<0.0001 One-way ANOVA). Similarly, LPS and fMLP were as effective (if not more) as prophylaxis vancomycin in controlling USA300 MRSA (10⁶ CFU) infection in the implant model (Fig 2C). The LPS results were particularly intriguing given that LPS (which is a component of Gram-negative pathogens), was able to enhance antimicrobial defenses against a USA300 which is a Gram positive pathogen. This finding supports that the immune system controls various infections regardless of their origin, provided that the immune system components are mobilized to the site of infection in a timely manner. CCL3 and GM-CSF are also tested using the implant model of infection. Here, we tested CCL3 and GM-CSF in the wound model of infection in C57B mice. Data indicated that topical addition of CCL3 was as effective in controlling PA103 infection (both at 10³ and 10⁶ CFU) as prophylaxis tobramycin (Fig 2B, data for 10³ CFU are not shown). Fig. 3A and 3B show that CCL3 is effective against PA103 infection and the combination of CCL3 and tobramycin enhanced the effectiveness in controlling PA103 infection. Fig. 6A and 6B show that IL-1β is effective in controlling both P.aeruginosa and MRSA infections in normal wound models.

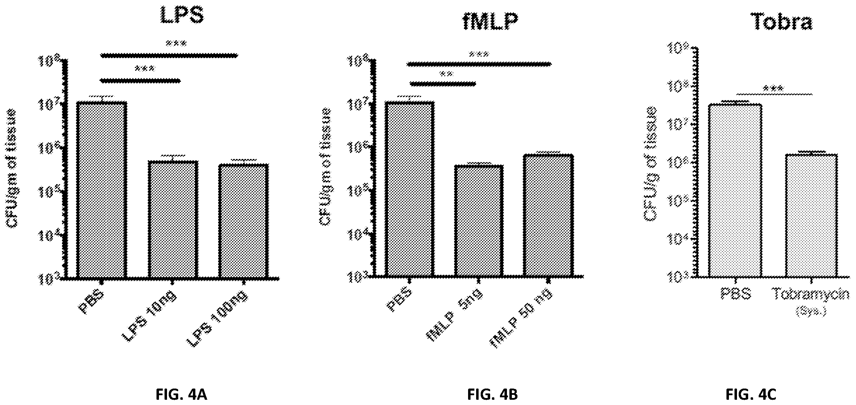

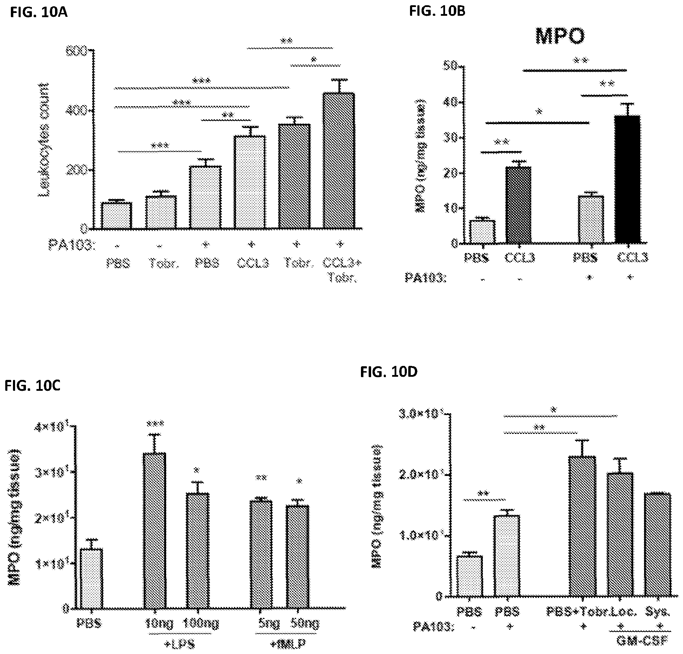

Systemic GM-CSF was also able to significantly reduce PA103 infection in wounds as compared to PBS-treated wounds, but it was not as effective as CCL3 or tobramycin (Fig 2B). LPS and fMLP significantly improved infection control against *P. aeruginosa* in normal wounds as shown in Figs. 4A and 4B. LPS and fMLP also improved infection control in the implant model against S. aureus. Finally, LPS, fMLP, and CCL3 significantly improved infection control against P. *aeruginosa* in diabetic wounds infected with PA103, although CCL3 was significantly more effective than LPS or fMLP in diabetic animals (Fig 2D). We next examined the impact of CCL3 topical treatment on leukocyte migration by H&E staining, and on leukocyte activation by myeloperoxidase (MPO) measurements by ELISA. (MPO is primarily used as a marker for neutrophil activation but it can also be expressed to lesser extent in macrophages). Consistent with our hypothesis, addition of topical CCL3 to C57B wounds resulted in significant increases in leukocytes' migration and activation in the wound, regardless of infection (Fig 10A, 10B, N=4 animals/group, **p*<0.01, ***p*<0.001, ****p*<0.0001, One-way ANOVA).

Assessment of CCL3-based approaches (single vs. combination with prophylaxis antibiotics) in controlling SSI.

A logical assumption is that prophylaxis antibiotics not only eradicate infective agent at the site of infection, but they also prevent systemic infection. However, our data show (FIG. 15A and 15B) that 24h after the cessation of standard prophylaxis antibiotic therapy, considerable number of bacteria still remain both at the site of infection (tissue and implant) and at a distal site in the spleen, indicating potential systemic infection. The immunomodulators we tested in our studies, were all as effective if not better than systemic antibiotics in controlling infection both on the implant and in the tissue surrounding the implant (Fig 2A-C), though we did not evaluate their impact on systemic infections.

We will evaluate the effectiveness of CCL3 immunomodulator, alone or in combination with bactericidal and bacteriostatic prophylaxis antibiotics, in controlling short- and long-term local and systemic infections, using the wound and the implant infection models with MRSA and P. *aeruginosa.* C57B will be treated with CCL3 alone or in combination with tobramycin (Tob), vancomycin (Vane), or Linezolid (Lin) prophylaxis antibiotics. At 5h and on days 1, 5, and 10 following infection, implants, tissues surrounding the implants or wound tissues (surgical sites), as well as, spleen, kidney, and liver (distant organs) will be collected and assessed for bacterial titers by CFU counts, to evaluate the short- and long-term effectiveness of CCL3 in controlling local and systemic infections.

CCL3-based therapies vs. prophylaxis antibiotics in preventing bacterial reservoir at surgical sites *in vivo*

Surgical site infection rates following elective implant removal is substantially higher (12.2%-14%) than SSI rates associated with implant surgeries (-3.8%), despite the fact that removal of orthopedic implants is considered a clean surgical procedure without skin contamination or local infection. This curious finding raises the question as to the sources of infection in these clean surgical procedures. Because prophylaxis systemic antibiotics are not able to destroy all bacteria (Fig 15A), the surviving bacteria may form biofilms on implants or in tissues surrounding implants, which protect them from immune defenses and also act as reservoirs for bacteria in subsequent infections upon elective implant removal or revision surgeries. Immunomodulators may be more effective in reducing these bacterial biofilm reservoirs *in vivo*, as they mobilize and direct host immune responses toward the surgical sites before these bacteria have the opportunity to form biofilms. We found significantly higher bacterial loads on implants in animals which were treated with prophylaxis vancomycin or tobramycin as compared to implants treated with immunomodulators (Fig 15A & Figs 2A & 2C). We will collect implants and tissue surrounding implants on days 5 and 10 and evaluate them for the presence of biofilm bacteria by bacterial load assessment, as discussed above (to account for viable bacterial loads on implants); and for biofilm assessment by Lectin and DNA staining and confocal microscopy and by transmission or scanning electron microscopy. These studies will address this critical knowledge gap in our understanding of potential bacterial reservoirs that can form at surgical sites after cessation of prophylaxis antibiotics. In these studies, PA103 (a non-flagellated non-biofilm forming *P*. *aeruginosa* strain) serves as a negative control for PAO1 (a flagellated biofilm-producing *P*. *aeruginosa* strain). Immunomodulators may be applied to the implants to reduce the bacteria and to decrease the infection in removal or revision surgeries.

### Immunomodulator Therapies in uninfected diabetic wounds

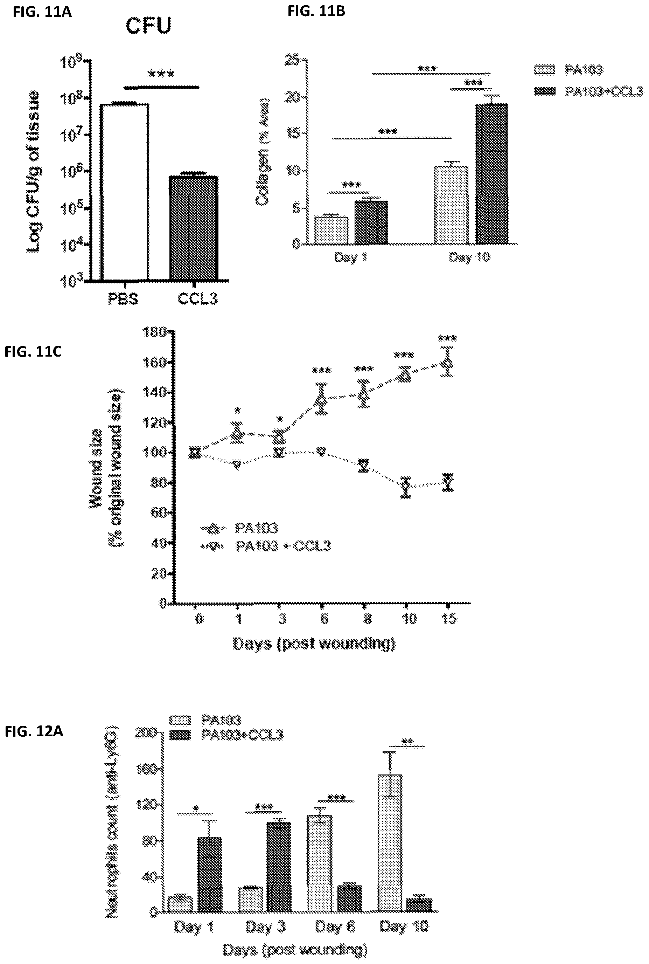

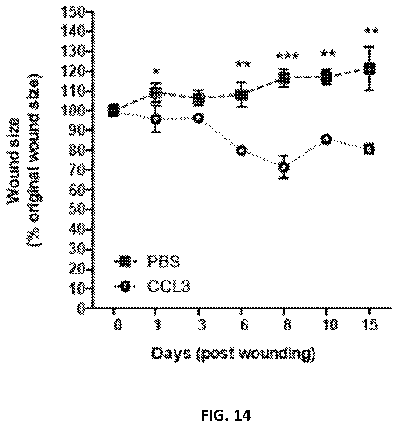

As discussed above immunomodulatory therapies enhance infection control in both normal and diabetic models. FIGS. 11A-11C illustrate that CCL3 increases infection control (11A) increases collagen deposition (11B) and enhances wound healing (11C) in diabetic wounds. Immunomodulators may also be used to enhance wound healing in uninfected diabetic wounds. As shown in FIG. 14, CCL3 enhances wound healing in diabetic uninfected wounds.

### Materials and Methods

The references to the methods of treatment by therapy or surgery in the examples of this description are to be interpreted as references to compounds and medicaments of the present invention for use in those methods.

### Implant and wound surgery

Implant surgery and wound surgery and surgical site infection are performed according to the following method. On the day of surgery, all mice are anesthetized by an intraperitoneal injection of a ketamine/xylazine mixture (90 mg/kg ketamine + 5 mg/kg xylazine for mice). The back of the animals is shaved using an electric shave and scrubbed with alcohol swabs 3 times, a topical antiseptic solution (Hibiclens; chlorhexidine gluconate) is applied to the skin. The surgical procedures are performed with sterile instruments, sterile surgical gloves, and aseptic techniques as follows. For wound surgical procedure, a sterile biopsy punch (5-7 mm diameter) is used to punch through the full thickness of the back skin below the shoulder blades. Four 5-7 mm skin wounds are made on the back of each mouse. Wounds placed in this area cannot be reached by the animals and therefore are protected from self-licking. For implant surgery, a 4-mm long lateral skin incision is made on the upper back, and the skin is retracted. The space above the muscle is opened using blunt dissection with fine forceps, to create a rostral fascia indentation. Then, immunomodulators at indicated concentrations (in a 20 µL volume) or PBS control are injected into the fascia indentation. After a 1 min wait for fluid to be absorbed, a disc implant, (1.87 mm diameter, 0.68 mm thick (Part# VP 724F-1)), is inserted in the fascia indentation. Infection is carried out by addition of 10₆ CFU (resuspended in 20 µl PBS) of bacteria, for example USA300, (a relevant MRSA strain in surgical site infections), or PA103 (a clinical P. *aeruginosa* isolate which we have shown to cause infection and wound damage in diabetic wounds ₁₆₂), or PA103::Tob_{R},or *S*. *aureus* directly into the wound (wound model), or into fascia indentation (implant model) with a micropipette. For implant surgery, the fascia indentation is left open, while the skin is closed with 4-0 nylon sutures. The fascia above the back muscles in mice is too thin and fragile to allow it to be sutured closed around the implant.

Inflammation-incompetent mice are generated and maintained by the intraperitoneal injections of anti-mouse anti-Gr1 (Ly6-G) antibody, shown to effectively deplete circulating phagocytic cells such as PMN and clodronate containing liposomes (Moleculaire Celbiologie, The Netherlands), which effectively eliminates macrophages 48 hours prior to infection. The maximum PMN and Macrophage cell depletion occurs 24-to 48 hrs post injection respectively and the treatment remains effective until 5 days post treatment. The treatment includes of 200µl (100µg) of anti-mouse, anti-Gr1 (Ly6-G) antibody and 200 µl of clodronate liposome (injected intraperitoneally into each mouse. To determine the efficacy of phagocytic cell depletion treatment, 24 hours after injection of the antibodies 3 mice from each panel will be anesthetized by an intraperitoneal injection of a ketamine/xylazine mixture (90 mg/kg ketamine plus 5 mg/kg xylazine). 200µl of blood will then be drawn from the retro-orbital plexus of these mice, using capillarty tube, mixed with 50 µl heparin. Cells will be stained with APC-conjugated Gr1-specific antibody, FITC-conjugated CD11b neutrophils-specific antibody, and PE-conjugated F4/80 macrophage specific antibodies for 20 min on ice and analyzed for their macrophage and neutrophils contents by flow cytometry, using Becton Dickinson FACScan as we have described ₁₁₀. All mice will be anesthetized by an intraperitoneal injection of a ketamine/xylazine mixture (90 mg/kg ketamine plus 5 mg/kg xylazine). (For inflammation-incompetent mice, the procedure will begin 48 hrs after antibody and clodronate treatments). At indicated timepoints after infection, animals will be euthanized with carbon dioxide in a closed chamber. Under aseptic conditions in a biological hood, implants, tissues surrounding implants, wounds, spleen, liver, and kidney will be collected. Half of these tissues will be fixed in buffered formalin, embedded in paraffin, sectioned, and stained with H&E, or IHC. Histological analyses will be performed. The other half, will be weighed and placed in a 47-mm sterile Petri dish and processed for CFU count determination by serial dilution and plating. The implant site will be opened, and the metal disc will be removed and placed in a sterile microcentrifuge tube for analyses as described in the proposal. The tissue capsule around the implant site will then be removed and processed for analyses.

### Materials

Tissue-Tek O.C.T. (Sakura); Lysing Matrix D (MP Biomedicals); Collagenase (Roche Diagnostics), Hematoxylin and eosin (H&E); DNase 1 (Sigma-Aldrich); Hyluronidase, Streptozotocin (Sigma-Aldrich); Trizol (Invitrogen); CD45 antibody (eBioscience, Inc); Ly6C; Ly6G; CD11b; CD3 (BioLegend); CD68; GAPDH (Sigma-Aldrich). Ly6G and CD68 (Abcam); Primer pairs for GAPDH were from Qiagen. Masson's trichrome stain (Sigma-Aldrich); Picrosirius red (Abcam); Fibronectin (Chemicon International); Insulin slow-release capsules (Linplant; LinShin, Toronto, Canada) CCL3 (R&D Systems) fMLP (Sigma-Aldrich); II-1β ELISA (R&D Systems); TNF-α ELISA (R&D Systems).

## Claims

1. An immunomodulator for use in the treatment of a bacterial infection in a subject, comprising administering a therapeutically effective amount of the immunomodulator to the subject;
wherein the immunomodulator comprises Chemokine (C-C motif) ligand 3 (CCL3) having an Asp>26Ala substitution in the mature form of human CCL3 of (SEQ ID NO: 2); and wherein the bacteria comprises Methicillin-resistant *Staphylococcus aureus* (MRSA) or *Pseudomonas aeruginosa.*

2. The immunomodulatory for use according to claim 1, wherein the infection is in a wound in the subject or the infection is a surgical site infection.

3. The immunomodulator for use according to claim 1 or 2, further comprising administering an antibiotic.

4. The immunomodulator for use according to any one of claims 1-3, wherein the subject is a diabetic.

5. The immunomodulator for use according to any one of claims 1-4, wherein the immunomodulator is applied topically or systemically.

6. The immunomodulator for use according to claim 4, wherein the immunomodulator is applied topically to a diabetic wound after wound debridement.

## Patentansprüche

1. Immunmodulator zur Verwendung bei der Behandlung einer bakteriellen Infektion in einem Subjekt, umfassend das Verabreichen einer therapeutisch wirksamen Menge des Immunmodulators an das Subjekt;
wobei der Immunmodulator Chemokin (C-C-Motiv) Ligand 3 (CCL3) mit einer Substitution Asp>26Ala in der reifen Form von menschlichem CCL3 von (SEQ ID NO: 2) umfasst; und wobei die Bakterien Methicillin-resistenten *Staphylococcus aureus* (MRSA) oder *Pseudomonas aeruginosa* umfassen.

2. Immunmodulator zur Verwendung nach Anspruch 1, wobei die Infektion in einer Wunde in dem Subjekt vorliegt oder die Infektion eine Infektion an einem chirurgischen Eingriffsort ist.

3. Immunmodulator zur Verwendung nach Anspruch 1 oder 2, ferner umfassend das Verabreichen eines Antibiotikums.

4. Immunmodulator zur Verwendung nach einem der Ansprüche 1-3, wobei das Subjekt Diabetiker ist.

5. Immunmodulator zur Verwendung nach einem der Ansprüche 1-4, wobei der Immunmodulator topisch oder systemisch verabreicht wird.

6. Immunmodulator zur Verwendung nach Anspruch 4, wobei der Immunmodulator topisch nach Wunddebridement auf eine diabetische Wunde aufgetragen wird.

## Revendications

1. Immunomodulateur pour une utilisation dans le traitement d'une infection bactérienne chez un sujet, comprenant une administration d'une quantité thérapeutiquement efficace de l'immunomodulateur au sujet ;
dans lequel l'immunomodulateur comprend le ligand 3 (motif C-C) de chimiokine (CCL3) ayant une substitution Asp>26Ala dans la forme mature de CCL3 humaine de (SEQ ID NO: 2) ; et dans lequel la bactérie comprend un *Staphylococcus aureus* résistant à la méthicilline (MRSA) ou *Pseudomonas aeruginosa.*

2. Immunomodulateur pour une utilisation selon la revendication 1, dans lequel l'infection se trouve dans une plaie du sujet ou l'infection est une infection de site chirurgical.

3. Immunomodulateur pour une utilisation selon la revendication 1 ou 2, comprenant en outre l'administration d'un antibiotique.

4. Immunomodulateur pour une utilisation selon l'une quelconque des revendications 1-3, dans lequel le sujet est diabétique.

5. Immunomodulateur pour une utilisation selon l'une quelconque des revendications 1-4, dans lequel l'immunomodulateur est appliqué de manière topique ou de manière systémique.

6. Immunomodulateur pour une utilisation selon la revendication 4, dans lequel l'immunomodulateur est appliqué de manière topique sur une plaie diabétique après débridement de la plaie.
